# EUROPEAN PATENT SPECIFICATION

(11) **EP 3 139 971 B1**
(45) Date of publication and mention of the grant of the patent: **19.08.2020**
(21) Application number: 15789496.5
(22) Date of filing: 05.05.2015
(51) Int. Cl.: A61L 27/34

(54) **COMPOSITIONS FOR SURFACE MINERALIZATION**
ZUSAMMENSETZUNGEN ZUR OBERFLÄCHENMINERALISIERUNG
COMPOSITIONS POUR MINÉRALISATION DE SURFACE

(30) Priority: 06.05.2014 US 201461989272 P
(43) Date of publication of application: 15.03.2017
(73) Proprietor: University of Massachusetts Medical School, Boston, MA 02110 (US)
(72) Inventor: SONG, Jie, Shrewsbury, MA 01545 (US); LIU, Pingsheng, Worcester, MA 01604 (US); AYERS, David C., Southborough, MA 01772 (US)
(74) Representative: Ter Meer Steinmeister & Partner
(86) International application number: PCT/US2015/029176
(87) International publication number: WO 2015/171564

(56) References cited:
- WO-A1-2014/085275
- US-A1- 2010 145 286
- LIU, P. ET AL.: "Modification of Ti6Al4V Substrates with Well-defined Zwitterionic Polysulfobetaine Brushes for Improved Surface Mineralization", APPL. MATER. INTERFACES, vol. 6, 14 May 2014 (2014-05-14), pages 7141-7152, XP002774943,
- LIU ET AL.: 'Sulfobetaine as a zwitterionic mediator for 3D hydroxyapatite mineralization.' BIOMATERIALS, [Online] vol. 34, no. 10, March 2013, ISSN 0142-9612 pages 2442 - 2454, XP055234563 ISSN: 0142-9612 Retrieved from the Internet: <URL:http://www.ncbi.nlm.nih.gov/pm/article s/PMC3557615/pdf/nihms432589.pdf> [retrieved on 2015-07-16]

## Description

### Technical Field of the Invention

The invention generally relates to materials and methods for medical implants and devices. More particularly, the invention relates to novel compositions and methods of surface mineralization for metallic implants and devices and the resulting enhancement of properties and performance in skeletal tissue engineering, orthopedic applications and dental care.

### Background of the Invention

Titanium and its alloys are extensively used in orthopedics and dentistry as implants due to their excellent mechanical properties, corrosion and wear resistance, and biocompatibility. (Long, et al. 1998 Biomaterials 19 (18), 1621-39; Hijon, et al. 2004 Chem. Mater. 16 (8), 1451-1455; Hijon, et al. 2005 Chem. Mater. 17 (6), 1591-1596; Sul, et al. 2002 Biomaterials 23 (2), 491-501.) Ti6A14V (also known as Ti-6Al-4V or Ti 6-4), for example, is the material of choice in total joint replacement implants. However, implant loosening due to the lack of adequate tissue-implant integration remains a significant clinical challenge in total joint replacement. Lack of adequate osteointegration of the metallic implant with surrounding skeletal tissues could lead to early implant failure. (Bandyopadhyay, et al. 2010 Acta Biomater. 6 (4), 1640-1648.)

Numerous attempts have been made to address this challenge, including generating porous implant surfaces or increasing surface roughness to facilitate bone in-growth, introducing osteoconductive bioceramic coatings (*e.g*., hydroxyapatite, HA) to promote bone cell attachment, and locally delivering osteogenic growth factors (*e.g*., rhBMP-2) on implant surfaces to stimulate its osteointegration. (Lopez-Heredia, et al. 2008 J. Biomed. Mater. Res. A 85A (3), 664-673; Bose, et al. 2012 Trends Biotechnol. 30 (10), 546-554; Aparicio, et al. 2011 Mat. Sci. Eng. C-Mater. 31 (2), 320-324; Aparicio, et al. 2003 Biomaterials 24 (2), 263-273; Svehla, et al. 2002 J. Arthroplasty 17 (3), 304-311; Narayanan, et al. 2008 J. Biomed. Mater. Res. B 85B (1), 279-299; Alghamdi, et al. 2012 Tissue Eng. Pt. B-Re. 18 (5), 383-395; Bae, et al. 2012 J. Control. Release. 160 (3), 676-684; Bose, et al. 2012 Acta Biomater. 8 (4), 1401-1421.)

One commonly used commercial technique for ceramic coating is plasma spray deposition, which generates a non-uniform calcium phosphate (CaP) layer up to several hundred micrometers thick. CaP layers deposited by plasma spray deposition contain a mixture of amorphous and crystalline composites that tend to prematurely dissolve and delaminate *in vivo.* (Stigter, et al. 2002 Biomaterials 23:4143; Xue, et al. 2004 Biomaterials 25:415; Daugaard, et al. 2010 J Biomed Mater Res A 92:913; Blackwood, et al. 2010 J Biomed Mater Res A 93:1551.) Furthermore, this "line of sight technique" is not suitable for coating porous substrates. One strategy for improving the crystallinity of the ceramic coating following plasma spray deposition has been to heat-treat the mineralized substrate at very high-temperatures, which could negatively impact the substrate's mechanical properties.

Other conventional approaches towards coating metallic implant with bioceramics involve either electrochemical deposition or incubation of substrates in simulated body fluid (SBF), which generate a CaP surface mineral layer from a supersaturated ion solution at physiological temperatures via prolonged incubation. (Daugaard, et al. 2010 J Biomed Mater Res A 92:913; Blackwood, et al. 2010 J Biomed Mater Res A 93:1551; Cai, et al. 2010 Acta Biomater 6:2314; Chen, et al. 2009 Acta Biomater 5:1808; Eliaz, et al. 2009 Acta Biomaterialia 5:3178; Kizuki, et al. 2010 Acta Biomater 6:2836; Park, et al. 2010 Acta Biomater 6:2843.) The thick coatings, however, lack adequate adhesion to the substrate. For instance, they do not survive supersonication and tend to peel off during treatment.

WO2014/085275 A1 relates to surface coatings having surface polymer brushes with zwitterionic and antibiotics-conjugated side chains and synergistic anti-fouling and bactericidal properties. The surface coatings may be prepared using highly efficient surface-initiated "living" polymerization. Liu, P. et al.: "Modification of Ti6AI4V Substrates with Well-defined Zwitterionic Polysulfobetaine Brushes for Improved Surface Mineralization", Appl. Mater. Interfaces, vol. 6, 2014, pp. 7141-7152 describes the covalent grafting of zwitterionic poly(sulfobetaine methacrylate) (pSBMA) brushes on a Ti6A14V substrate to promote the surface mineralization of hydroxyapatite with enhanced surface mineral coverage and mineral-sSubstrate interfacial adhesion. Liu et al.: "Sulfobetaine as a zwitterionic mediator for 3D hydroxylapatite mineralization", Biomaterials, vol. 34, no. 10, March 2013, pp. 2442-2454 describes the use of cytocompatible zwitterionic ligands to enable 3-diemnsional *in vitro* mineralization of hydroxyapatite (HA) across covalently crosslinked hydrogels. US 2010/145286 A1 relates to substrates having grafted thereto one or more non-fouling materials. The non-fouling, polymeric material can be grafted to a variety of functionalized substrate materials, particularly polymeric substrates and/or polymeric undercoatings immobilized on a substrate.

It is strongly desired that novel approaches to adequate tissue-implant integration be developed that provide a robust, reliable and biocompatible osteointegration of the metallic implant.

### Summary of the Invention

Disclosed herein is a novel approach where zwitterionic brushes (*e.g*., of poly(sulfobetaine methacrylate) or pSBMA) are covalently grafted on the surface of titanium or its alloy substrates (*e.g*., Ti6A14V) or ceramic substrates to promote surface-mineralization of hydroxyapatite with enhanced surface mineral coverage and mineral-substrate interfacial adhesion. The zwitterionic surface brushes, capable of attracting both cationic and anionic precursor ions during hydroxyapatite-mineralization, significantly increase the surface mineral coverage (*e.g*., by 39% or greater) and significantly reinforce the attachment of the surface apatite crystals on the titanium alloy substrate which withstood supersonication treatment.

The unique approach disclosed herein - covalently grafting zwitterionic polymer brushes to metal alloy surfaces and subsequently templating the nucleation and growth of mineral coating, either *in vitro* or *in vivo* - enables revolutionary enhancement of the properties and performance of metallic or ceramic implants in skeletal tissue engineering and orthopedic and dental care.

In one aspect, the invention generally relates to a surface layer on a substrate having a structurally integrated mineral grown from a zwitterionic polymer template, wherein the zwitterionic polymer is covalently linked to the substrate surface.

Further described is a device, or component thereof, having a surface covalently bonded thereto a zwitterionic polymer and a layer of a structurally integrated mineral grown from the zwitterionic polymer as template. The zwitterionic polymer comprises a repeating unit having structure of: 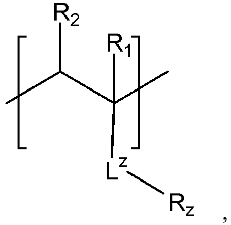 wherein
- R₁: is a hydrogen, alkyl, alkyloxy;
- R₂: is a hydrogen, (C₁-C₁₅) alkyl, (C₁-C₁₅) alkyloxy;
- L^{z}: is a linking group; and
- R_{z}: is a pendant group comprising a zwitterionic group.

### Brief Description of the Drawings

**FIG. 1****.** Well-controlled ATRP of zwitterionic SBMA carried out in 10 wt% HMImCl in TFE. **a,** Monomer conversion (%) and conversion index ln([M]/[M]0) as a function of reaction time at rt (squares) and 60 °C (stars); **b,** Molecular weight and polydispersity index (PDI) as a function of monomer conversion (%) at rt (squares) and 60 °C (stars). [SBMA] = 1 M, [SBMA]:[EBiB]:[CuBr]:[BPY] = 100:1:1:2. c, GPC traces ofpSBMA with different degree of polymerizations (DPs) prepared in 10 wt% HMImCl/TFE at 60 °C (PDI & Mn summarized in **Table 1**).

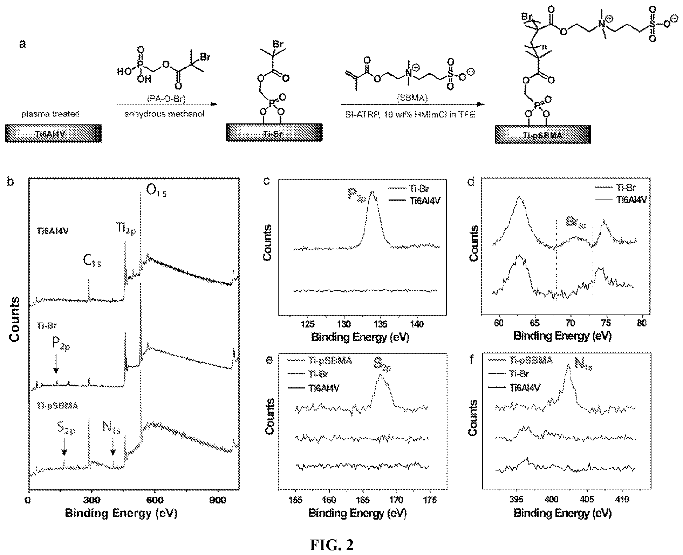

**FIG. 2****.** Grafting of pSBMA brushes from the Ti6A14V substrate, **a,** Schematic illustration of the grafting of pSBMA brushes from the Ti6A14V substrate by SI-ATRP. **b,** XPS survey scans on the Ti6A14V surfaces before and after immobilization of anchorable initiators and subsequent SI-ATRP. c, high resolution scans of P_{2P} of Ti6A14V and Ti-Br surfaces. **d**, high resolution scans of Br_{3d} of the Ti6A14V and Ti-Br surfaces; the binding energy range of Br_{3d} was indicated by the red dash lines. **e**, high resolution scans of S_{2P} of Ti6A14V, Ti-Br and Ti-pSBMA surfaces, **f,** high resolution scans of N₁ₛ of Ti6A14V, Ti-Br and Ti-pSBMA surfaces.
**FIG. 3****. a,** Water contact angles on Ti6A14V, Ti-Br, and the Ti-pSBMA surfaces with different DPs of grafted pSBMA brushes (n=3). All differences are significant (P < 0.05, one-way ANOVA multiple comparison) unless denoted as ns (not significant). **b**, Florescent micrograph, and **c,** Fluorescent intensity line plot showing substantially reduced non-specific absorption of fluorescein-conjugated BSA on the Ti6A14V substrate upon surface grafting of pSBMA (DP=200).

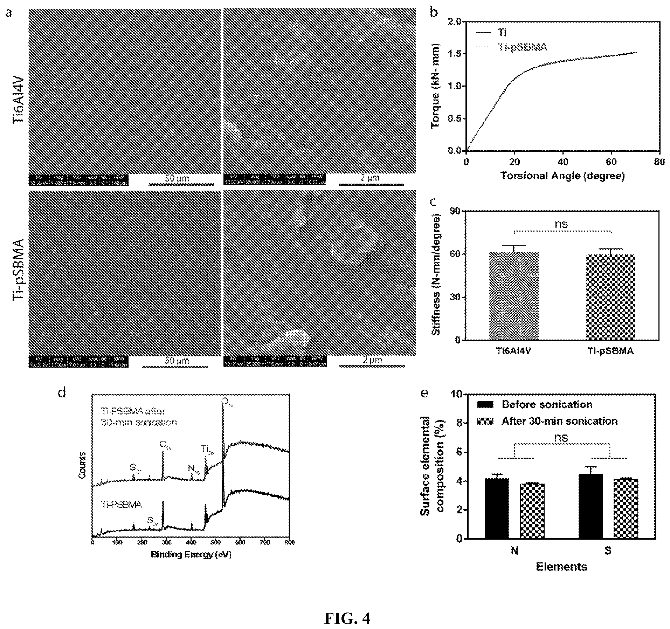

**FIG. 4****.** Surface morphology and mechanical property of the Ti6A14V substrates before and after grafting pSBMA brushes and the stability of the pSBMA brush coating. **a**, SEM micrographs of Ti6A14V and Ti-pSBMA surfaces. **b**, Torque-displacement curves of of Ti6A14V and Ti-pSBMA substrates, **c,** Torsional stiffness of Ti6A14V and Ti-pSBMA substrates (n = 3). The difference was not significant (P > 0.05, Student's t-test). **d**, XPS survey scans of the Ti-pSBMA surfaces before and after 30-min ultrasonication in TFE. **e,** N and S elemental contents (determined by XPS high resolution scans of N₁ₛ and S₂ₚ) on the Ti-pSBMA substrates (n = 3) before and after 30-min ultrasonication in TFE. No significant differences detected (P > 0.05, two-way ANOVA multiple comparison).
**FIG. 5****. a.** GPC traces of pSBMA cleaved from Ti-pSBMA (red) and the free pSBMA formed in solution before (black) and after acid treatment (blue). **b**. ¹H NMR spectra of the free pSBMA formed in solution before (black) and after acid treatment (blue).

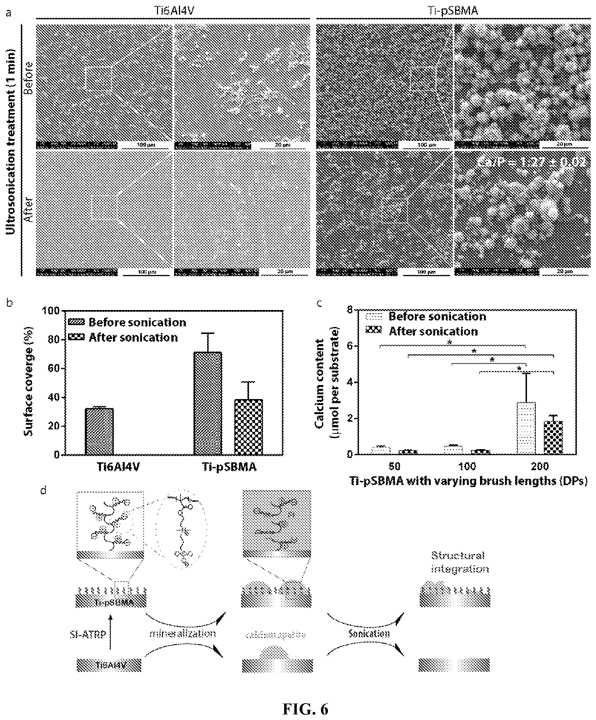

**FIG. 6****.** Mineralization on Ti6A14V substrates with and without surface-grafted pSBMA brushes. **a**, SEM micrographs of the mineralized substrates before and after a 1-min ultrasonic treatment. **b**, Surface mineral coverage on the substrates as determined by ImageJ (n = 7). All differences are significant (P < 0.05, two-way ANOVA). **c**, Ca²⁺ content on the mineralized Ti-pSBMA substrates (n = 3) as a function of degree of polymerization (DPs) of the pSBMA brushes. Differences are not significant (P > 0.05, two-way ANOVA) unless denoted as asterisk (*). **d**, Schematic illustration of surface mineralization on the pristine Ti6A14V vs. on that surface-grafted with pSBMA brushes.
**FIG. 7****.** Mineralization on a porous Ti6A14V hip stem surface-grafted with pSBMA (DP=200). **a**, Photograph of a Taperloc® Complete Hip Stem prior to any surface treatment. **b**, SEM micrograph of the porous implant surfaces before (left) and after SI-ATRP coating and subsequent mineralization (right). **c**, EDX spectrum of the surface calcium apatite minerals.
**FIG. 8****.** Cell viability of rat MSCs cultured in 24-well culture plate (n=3) in the presence of Ti6A14V, Ti-pSBMA, and mineralized Ti-pSBMA substrates. Differences between substrates at a given time point are not significant (P > 0.05, two-way ANOVA multiple comparison) unless denoted by an asterisk (*).
**FIG. 9****.** Synthetic scheme for initiator PA-O-Br.

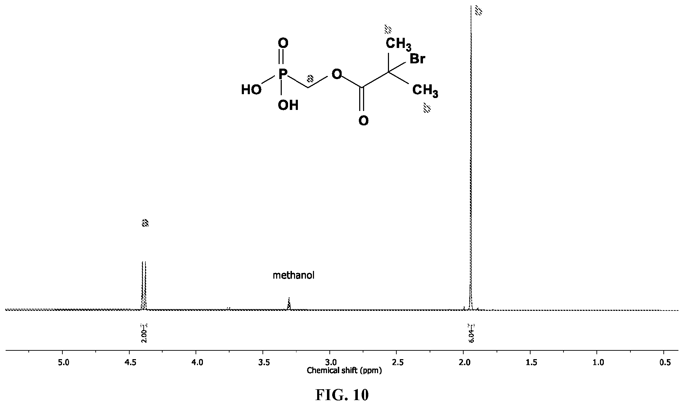

**FIG. 10****.** ¹H NMR spectrum of ATRP initiator PA-O-Br.

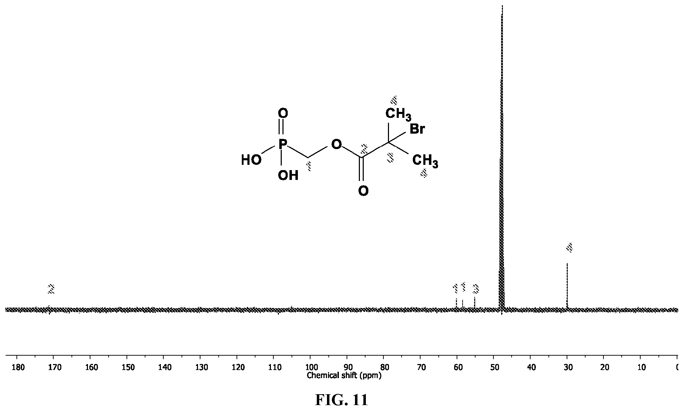

**FIG. 11****.** ¹³C NMR spectrum of ATRP initiator PA-O-Br.

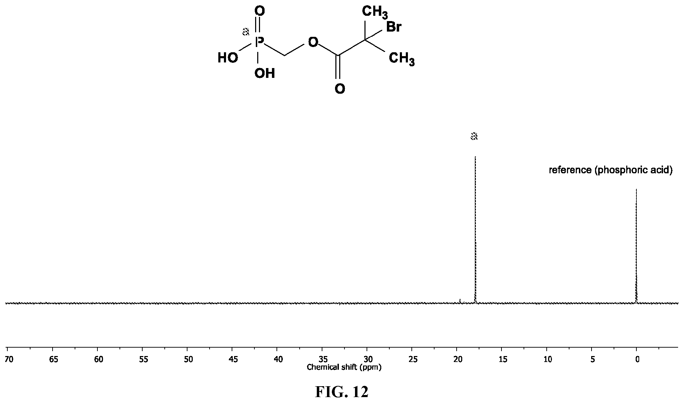

**FIG. 12****.** ³¹P NMR spectrum of ATRP initiator PA-O-Br.
**FIG. 13****.** Mass spectrum of ATRP initiator PA-O-Br.
**FIG. 14****.** Kinetics of the solution ATRP of SBMA in TFE at room temperature. [SBMA]:[EBiB]:[CuBr]:[BPY]=100:1:1:2.
**FIG. 15****.** GPC curves of the pSBMA polymers obtained from ATRP carried out in TFE/HMImCl at (a) room temperature *vs* (b) 60 °C. [SBMA]:[EBiB]:[CuBr]:[BPY]=100:1:1:2

### Detailed Description of the Invention

The invention provides a novel, simple and robust strategy for achieving significant enhancement of performance of metallic or ceramic implants. For example, substantial improvement in skeletal tissue engineering and orthopedic and dental care is enabled via covalently grafting of zwitterionic polymer brushes and subsequently templating the nucleation and growth of mineral coating to metal alloy or ceramic surfaces, either *in vitro* or *in vivo.*

Achieving adequate structural integration between the surface minerals and underlying metallic substrates has been a significant challenge for metallic or ceramic orthopedic/dental implants. Introduction of bioactive minerals to implant surfaces is particularly attractive as it could simultaneously confer osteoconductivity to improve cellular attachment, drug delivery capability, and long-term biocompatibility due to the bone mineral-like compositions. (Bose, et al. 2012 Acta Biomater. 8 (4), 1401-1421; Liu, et al. 2011 Acta Biomater. 7 (9), 3488-3495; Xu, et al. 2009 J. Orthopaedic Res. 27 (10), 1306-1311.)

Plasma spray of calcium apatite and *in vitro* heterogeneous mineralization employing various mineralization conditions have been utilized to create surface mineral coatings to metallic implants. (Liu, et al. 2011 Acta Biomater. 7 (9), 3488-3495; Filion, et al. 2011 Tissue Eng. Pt. A 17 (3-4), 503-511; Park, et al. Biomaterials: An introduction. 3rd ed.; Springer: New York, 2007**;** Sun, et al. 2001 J. Biomed. Mater. Res. 58 (5), 570-592; Kokubo, et al. 2006 Biomaterials 27 (15), 2907-2915.)

While the plasma spray technique has tremendous advantage in terms of its facile control of the thickness of the bioceramic coating applied to the implant, the heterogeneous mineralization approach has a unique advantage. When templated by proper mineral nucleation sites presented on the metallic implant surfaces, the heterogeneous mineralization approach is beneficial to not only the pre-implantation generation of osteoconductive coating, but also *in vivo* osteointegration during the dynamic implant surface remodeling (which could resorb the mineral coating, exposing underlying metal surfaces) post-implantation.

A major barrier is the inadequate adherence of the surface minerals to the metallic substrate due to sub-optimal choice/presentation of surface mineral nucleation sites (*e.g.,* often the surface oxides are utilized for templating the mineralization). (Liu, et al. 2011 Acta Biomater. 7 (9), 3488-3495.) Mediators of heterogeneous mineralization and their robust presentation on the metallic implant surface with controlled surface densities, as disclosed herein, are highly critical to successful commercial implementation of the heterogeneous mineralization approach.

Recently studies have shown that synthetic zwitterionic sulfobetaine hydrogels, possessing oppositely charged residues to attract both precursor cations and anions, could template extensive heterogeneous HA-mineralization throughout the 3-dimensional scaffold with excellent structural integration. (Liu, et al. 2013 Biomaterials 34 (10), 2442-54.) In addition, zwitterionic materials, well known for their low-fouling nature and biocompatibility, have been widely explored for applications as blood compatible materials and DNA and protein delivery vehicles. (Banerjee, et al. 2011 Adv. Mater. 23 (6), 690-718; Chen, et al. 2010 Polymer 51 (23), 5283-5293; Ishihara, et al. 1998 J. Biomed. Mater. Res. 39 (2), 323-330; Yan, et al. 2007 Sci. China. Ser. B 50 (5), 660-664; Nakaya, et al. 1999 Prog. Polym. Sci. 24 (1), 143-181; Liu, et al. 2010 J. Membr. Sci. 350 (1-2), 387-394; Jiang, et al. 2010 Adv. Mater. 22 (9), 920-952; Andrew, et al. 2012 Nat. Chem. 4 (1), 59-63.)

Surface-initiated atom transfer radical polymerization (SI-ATRP) is a well-established method for grafting uniform functional polymer brushes from various substrates. (Barbey, et al. 2009 Chem. Rev. 109 (11), 5437-5527.) By covalently tethering initiators to the surface of interest, ATRP of vinyl monomers could be triggered from the surface to generate well-controlled functional polymer brushes via the living radical polymerization. Silane coupling agents have long been used for tethering to titanium substrates. (Wang, et al. 2010 J. Polym. Sci. Pol. Chem. 48 (8), 1782-1790; Ren, et al. 2011 Langmuir 27 (19), 12069-12073; Zhang, et al. 2006 Ind. Eng. Chem. Res. 45 (9), 3067-3073; Heysel, et al. 1995 Protein Sci. 4 (12), 2532-2544; Xiao, et al. 1997 J. Mater. Sci.-Mater. M. 8 (12), 867-872; Xiao, et al. 1998 Langmuir 14 (19), 5507-5516; Rezania, et al. 1999 Langmuir 15 (20), 6931-6939.) The Ti-O-Si bond formed, however, exhibit quite low hydrolytic stability. (Marcinko, et al. 2004 Langmuir 20 (6), 2270-2273.) In contrast, phosphonic acid-terminated small molecules have been shown to form stable Ti-O-P bonds on titanium surfaces with better surface coverage and improved hydrolytic stability under physiological conditions compared to silanes, and was previously utilized to successfully form self-assembled monolayers on the metallic surface. (Xiao, et al. 1998 Langmuir 14 (19), 5507-5516; Marcinko, et al. 2004 Langmuir 20 (6), 2270-2273; Lafond, et al. 2003 Chem. Mater. 15 (21), 4098-4103.)

It is disclosed and demonstrated herein that zwitterionic polymer coatings, when applied to titanium substrates with good bonding affinity (*e.g*., via covalent grafting), can promote effective heterogeneous nucleation and facilitate growth of calcium apatite minerals on the implant surface with superior interfacial affinity and improved implant osteointegration *in vivo.* As exemplified by the disclosure herein, robust chemistry and optimal SI-ATRP conditions have been developed that allow covalently tethering phosphonic acid-based initiators onto Ti6A14V substrates and grafting well-controlled zwitterionic poly(sulfobetaine methacrylate) (pSBMA) brushes from the Ti6A14V substrates.

Comparing to plasma spray method, the current approach leads to HA-surface mineralization without blocking or changing the surface porosity of metallic implants. It can be applied to a wide range of metals and alloys, particularly those commonly used as orthopedic implants including Ti6A14V, TiO₂, and tantalum, as well as porous surfaces. Additionally, the pSBMA-grafted surface exhibits anti-fouling properties in addition to outstanding ability to template mineralization *in vitro* and *in vivo.* Furthermore, the crystalline calcium apatite coating resulting from the surface mineralization can be exploited as a delivery vehicle for biological therapeutics (*e.g*., osteogenic growth factors and antibiotics). Mineralized Ti6A14V substrates show enhanced retention of rhBMP-2 compared to unmineralized substrates at least 7-days after loading. (Liu, et al. 2011 Acta Biomaterialia, 7, 3488-3495).

### Optimization of ATRP of SBMA

Unlike that of conventional polar vinyl monomers, the ATRP of zwitterionic sulfobetaine monomer tend to be poorly controlled in conventional polar solvents (including water) because the strong electrostatic interactions between the oppositely charged residues of the sulfobetaine monomers and polymers compromise their solubility, resulting in polymers with a broad molecular weight distributions (MWD). (Lee, et al. 1998 Polym. Gels Netw. 6 (6), 493-511; Jiang, et al. US20110105712 A1.) Recently, homogenous polymerization of sulfobetaine monomers was reported in fluoroalchohol/ionic liquid systems. The well-controlled polymerization mediated by ionic liquid HMImCl and the improved polymer solubility in TFE yielded high molecular weight (MW) polysulfobetaines (up to 305 kD in MW) with narrow MWD (PDI ~1.20). (Terayama, et al. 2011 Macromolecules 44 (1), 104-111.)

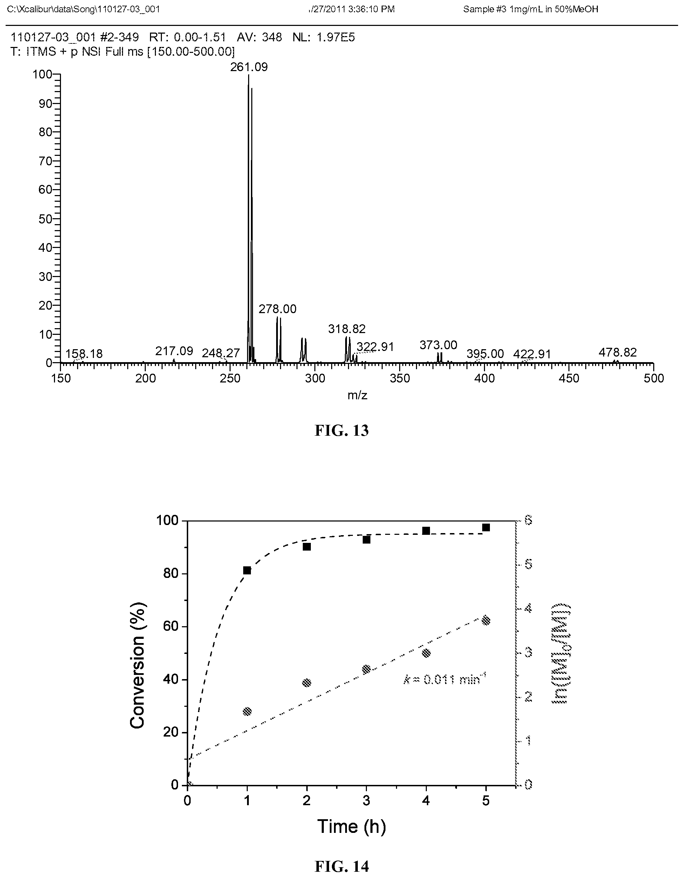

As disclosed herein, the TFE and TFE/HMImCl systems were investigated to identify optimal conditions for mediating well-controlled ATRP of SBMA. The ATRP of SBMA in TFE only was first carried out at room temperature with CuBr and BPY as the catalyst and catalyst ligand, respectively (**Table 1,** run 1). Over 80% of the monomers were converted into polymers within the first hour in a pseudo-first-order reaction kinetics, with an apparent propagation rate constant (*kₐₚₚ*) of 0.011 min⁻¹ (**FIG. 14**), supporting that the highly polar TFE enabled rapid monomer conversions. The MWD (Mₙ =19,382, PDI = 1.26) of the resulting pSBMA was narrower than those obtained in a water/methanol system (with PDI rapidly increasing from 1.26 to 1.47 when Mₙ increased from 4,764 to 90,340) where the electrostatic aggregation of pSBMA in the poor non-ionic solvent had likely compromised the exposure of the reactive ends for continued propagation. (Jiang, et al. US20110105712 A1.)

**Table 1. ATRP of SBMA in TFE or HMImCl/TFE (10 wt%) solutions**

| Run | DP | Solvent | temperature (C°) | reaction time (h) | conversion (%)^{a} | Mₙ (theo) (g/mol) | Mₙ (GPC) (g/mol) | PDI |
|---|---|---|---|---|---|---|---|---|
| 1 | 100 | TFE | 23 | 19 | > 99 | 27,851 | 19,382 | 1.26 |
| 2 | 100 | HMImCl/TFE | 23 | 21 | 93 | 26,175 | 15,321 | 1.14 |
| 3 | 50 | HMImCl/TFE | 60 | 18.5 | 99 | 14,023 | 12,643 | 1.13 |
| 4 | 100 | HMImCl/TFE | 60 | 6 | 90 | 25,337 | 16,823 | 1.14 |
| 5 | 200 | HMImCl/TFE | 60 | 21.5 | 93 | 51,154 | 25,478 | 1.17 |

| | | | | | | | | |
|---|---|---|---|---|---|---|---|---|
| ^{a} Determined by H NMR. | | | | | | | | |

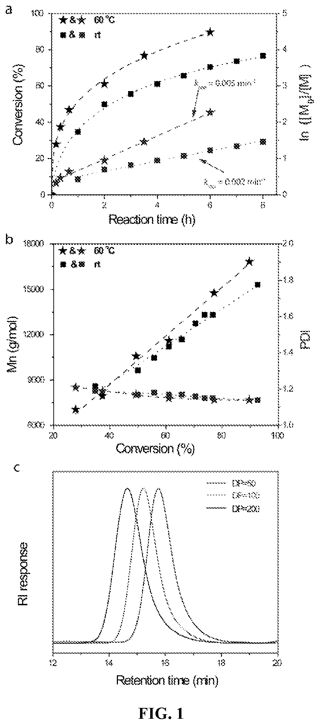

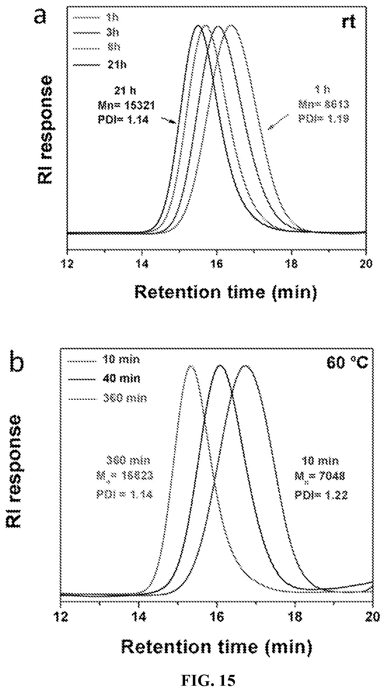

The ATRP of SBMA in TFE was significantly slowed with the introduction of ionic liquid imidazolium chloride (HMImCl, 10 wt% relative to TFE; **Table 1,** run 2). Only 35 % of the SBMA monomers were converted after 1 h and 93 % converted after 21 h in the present of HMImCl as revealed by ¹H NMR monitoring (**FIG. 1a****,** black squares). The *kₐₚₚ* decreased to one fifth of that in TFE alone (0.002 min⁻¹ *vs* 0.011 min⁻¹, **FIG.1a** blue square). However, the resulting pSBMA polymers with relative molecular weights up to 15,321 (**FIG. 1b**, black squares) remained narrowly dispersed (PDI = 1.14, **FIG. 1b****,** blue square, **FIG. 15a**), indicating that the polymerization proceeded without significant side reactions, active chain end termination or deactivation in the presence of the ionic liquid. Combined with the first-order kinetics revealed by linear plot of In(M₀/M) *vs* reaction time and the linear increase of MW over time (**FIG. 1b**, black square), these data support HMImCl as an effective mediator for controlled ATRP of sulfobetaine.

Further optimization of the reaction was carried out in TFE/HMImCl by elevating temperature while keeping other parameters unchanged (**Table 1,** run 4). It was showed that at 60 °C, 50 % of the monomers (vs 35% at room temperature) were converted in 1 h at an accelerated rate (*kₐₚₚ* of 0.005 min⁻¹, **FIG. 1a****,** star symbols). Despite the increased reaction kinetics, the ATRP remained well-controlled in the presence of the ionic liquid at 60 °C as characterized by the first-order kinetics (linear plot of In(M₀/M) *vs* reaction time, **FIG. 1a****,** blue star) and the linear increasing of the MW with narrow MWDs over time (PDI = 1.14 for Mₙ = 16,823, **FIG. 1b**, star symbols; **FIG. 15b**). Using the optimized polymerization medium of 10 wt% HMImCl in TFE and the elevated temperature of 60°C, well-controlled pSBMA polymers (PDI ≤ 1.17, relative MW up to 25,478) with targeted degrees of polymerizations (DPs) of 50, 100, and 200 were prepared (**Table 1,** run 3-5; **FIG. 1c**). This optimized solvent and temperature were then extended to the subsequent SI-ATRP for grafting pSBMA brushes from Ti6Al4V substrates.

### Grafting pSBMA brushes by SI-ATRP

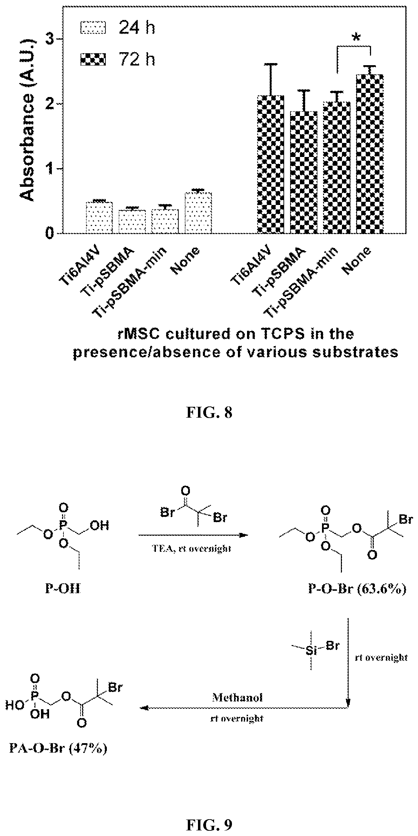

To create stably anchored SI-ATRP initiator on the Ti6A14V surface, phosphonic acid-terminated initiator PA-O-Br, synthesized in 3 steps (**FIG. 9**), was used to form stable Ti-O-P bonds. Air plasma-cleaned Ti6A14V substrates were soaked in 3 mM of PA-O-Br/ methanol solution for 24 h, and then annealed at 110 °C to immobilize PA-O-Br on the surface (**FIG. 2a**). The successful surface immobilization of the PA-O-Br on Ti6A14V was confirmed by the detection of the characteristic XPS signals for P₂ₚ (binding energy of 133.9 eV) and Br_{3d} (70.5 eV) core electrons that were absent from the unmodified Ti6A14V surface (**FIGs. 2b-2d**). Of note, the bromine signal detected on the Ti-Br surface was weaker than that of the phosphorus signal (0.37% of Br vs 6.39% of P), and did not match their 1:1 stoichiometric ratio in PA-O-Br. Although the high-temperature annealing process enhanced the bonding of PA-O-Br to the Ti6A14V substrate (*e.g.* only 0.18% of Br and 4.27% of P was detected by XPS from the Ti-Br substrate without annealing), it could have also potentially destabilized the terminal bromine residue. Meanwhile, it has also been reported that the C-Br bond was unstable under X-ray irradiation during XPS analysis, leading to similar observations. (Wasserman, et al. 1989 J. Am. Chem. Soc. 111 (15), 5852-5861; Zhang, et al. 2006 Langmuir 22 (24), 10072-10077.)

Grafting pSBMA brushes from the Ti-Br substrate via SI-ATRP was carried out in the optimized TFE/ HMImCl system at 60 °C in the presence of EBiB as the sacrificial free initiator. After extensive extraction of the modified substrates with TFE to move physically absorbed free polymers, the surface composition of the resulting Ti-pSBMA substrate was characterized by XPS. Two characteristic peaks corresponding to the core electrons of S₂ₚ (167.5 eV) and N₁ₛ (402.5 eV) and originating from the zwitterionic sulfobetaine side chains, were detected from the survey scans of Ti-pSBMA but not the Ti-Br substrates (**FIG. 2b**). The S and N signal intensities (4.02% of S vs 4.62% of N) revealed by the high-resolution scans (**FIGs. 2e & 2f**) approximated their 1:1 stoichiometric ratio, which, along with the significantly decreased Ti signals resulting from surface polymer coverage, supported successful grafting of the pSBMA brushes from the Ti6A14V substrate.

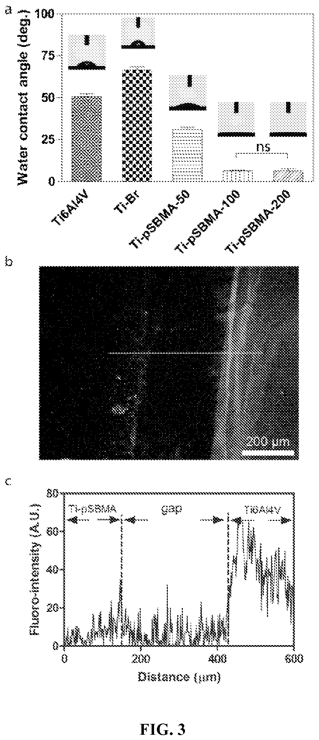

The grafting of superhydrophilic zwitterionic pSBMA brushes also resulted in the changes in surface wettability of the Ti6A14V substrate by water. As showed in **FIG. 3a****,** the immobilization of the Br-terminated initiator resulted in an increase in surface hydrophobicity (increase of the water contact angle from 50° to 62°) whereas subsequent grafting of pSBMA brushes with a DP of 50 sharply reduced the water contact angle by half. When pSBMA brushes with higher DP of 100 were grafted, the hydrophilicity of the surface that were more densely covered by the zwitterionic polymer brushes further increased, resulting in an exceptionally low water contact angel of ∼10°. However, increasing DP of the grafted pSBMA from 100 to 200 did not further decrease the surface water contact angles.

The successful grafting of pSBMA was verified by examining the anti-biofouling property of the surface modified with the zwitterionic polymer. (Yuan, et al. 2003 Colloid Surface B 29 (4), 247-256; Chang, et al. 2006 Langmuir 22 (5), 2222-2226; Liu, et al. 2009 Biomacromolecules 10 (10), 2809-2816; Smith, et al. 2012 Sci. Transl. Med. 4 (153).) As shown in **FIG. 3b**, whereas significant non-specific absorption of fluorescein-labeled BSA on Ti6A14V substrates was observed upon incubation of the substrate in the protein solution followed by copious rinsing, almost no absorption was detected from the substrate grafted with pSBMA brushes (Ti-pSBMA-200), which was further validated by the quantitative line plot analysis of the detected fluorescent intensities of both substrate surfaces (**FIG. 3c**). These data suggest that the grafted pSBMA brushes sufficiently covered the Ti6A14V surface and conferred the anti-fouling property characteristic of the zwitterionic polymer.

### Impact of grafting on the surface morphology, mechanical properties and stability of surface brushes

It was investigated whether/how grafting pSBMA brushes by SI-ATRP changes the surface morphology or compromises the mechanical property of the Ti6Al4V substrates. Low magnification (1,000 ×) SEM micrographs revealed similar polishing trails of the substrates with and without grafting of pSBMA brushes while the higher magnification (25,000 ×) SEM micrographs confirmed that grafting pSBMA by SI-ATRP did not induce significant changes in surface morphologies of the substrates on the micron scale (**FIG. 4a**). Torsional mechanical properties of the substrates remained largely unaffected upon grafting pSBMA as supported by the overlapping torque-displacement curves of Ti6Al4V and Ti-pSBMA substrates (**FIG. 4b**). No statistically significant difference in torsional stiffness of the substrates was detected (**FIG. 4c**). These observations support that the SI-ATRP process did not compromise the mechanical integrity of the Ti6A14V substrates.

To investigate the stability of the grafted pSBMA brushes, the Ti-pSBMA substrates were subjected to bath-sonication in TFE for 30 min (note that all substrates were extracted in TFE, a good solvent for free pSBMA, for 24 h to remove physically absorbed free polymers prior to sonication). The retrieved substrates were then rinsed with fresh TFE, vacuum-dried and subjected to XPS analysis. From the survey scans (**FIG. 4d**), the N and S signals associated with the pSBMA brushes were observed with similar intensities with and without the sonication of Ti-pSBMA. Furthermore, quantitation of the N and S elemental contents by high-resolution scans (**FIG. 4e**) confirmed that not only the absolute contents but also the stoichiometric ratio of these elements remained the same after the sonication treatment, supporting that the pSBMA brushes were stably grafted to the Ti6A14V substrate.

### Characterization of the surface-grafted pSBMA brushes

To characterize the MW and MWD of the grafted pSBMA brushes on the Ti6A14V surface, the Ti-pSBMA substrates were incubated in 2-M HCl aqueous solution. The cleaved polymer solution was neutralized with NaOH, desalted by dialysis and freeze-dried for GPC analyses. As shown in **Table 2,** the grafted pSBMA brushes exhibited a narrow MWD (PDI = 1.15, Mₙ = 17,246) similar to that of the free polymers formed with the sacrificial free initiator in the solution (PDI = 1.18, Mₙ = 19,710) from the same pot polymerization, supporting that a well-controlled SI-ATRP of SBMA was accomplished.

**Table 2. Properties of the solution pSBMA vs surface-grafted pSBMA cleaved from the Ti-pSBMA substrate**

| No. | polymer type | Mₙ (theo) (g/mol) | Mₙ (GPC) (g/mol) | PDI |
|---|---|---|---|---|
| a | free | 38,187 | 19,710 | 1.18 |
| b | free | 38,187 | 19,576 | 1.18 |
| c | brush | | 17,246 | 1.15 |

| | | | | |
|---|---|---|---|---|
| a, free polymer (initiated by with the sacrificial free initiators in solution). b, free polymer of a, but treated with the acidic cleavage solution (2-M HCl, 72 h). c, brush polymer cleaved by 2-M HCL (72 h) from the Ti-pSBMA substrate prepared in the same pot of SI-ATRP as those in a. | | | | |

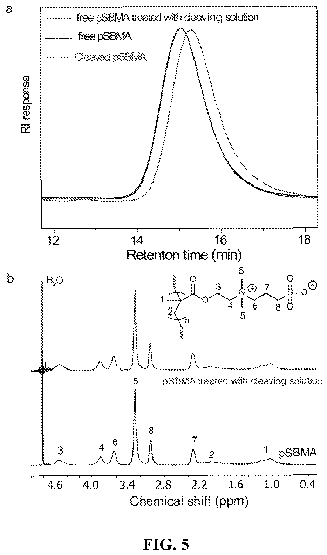

To examine whether the relatively lower MW of the grafted pSBMA compared to that of the free pSBMA (by 13%, **Table 2,** **FIG. 5a**) was due to the hydrolysis of the zwitterionic side chains during the acidic cleavage, the free pSBMA was subjected to the same acid treatment. GPC and 1H NMR analyses of the free pSBMA before and after the acid treatment revealed no difference in the polymer retention time, MW, PDI (**FIG. 5a****,** entries a & b in **Table 2**) or the proton integration ratio between the backbone methyl group (C-CH₃) and the side chain methyl group (N-CH₃) (**FIG. 5b**). Thus, the side chains of the pSBMA remained stable during the grafted brush cleaving process, and the lower MW observed with the grafted pSBMA was likely a result of the relatively slower kinetics of the SI-ATRP compared to that of the solution ATRP taking place in the same pot. The surface-bound initiator presented on Ti-Br substrate as well as the propagating reactive chain ends of the polymers grafted from the substrate have intrinsically reduced degrees of freedom compared to the free initiators and free propagating polymers in the solution, thus fewer chances to encounter monomers and consequently relatively slower propagation rate and lower MW obtainable in a given time. (Wang, et al. 1995 J. Am. Chem. Soc. 117 (20), 5614-5615; Matyjaszewski, et al. 2001 Chem. Rev. 101 (9), 2921-2990.) Indeed, although the addition of free initiators into the SI-ATRP system is a widely accepted method to determine the DP of solution polymer and extrapolate as that of the grafted polymer brush, actual differences between the free solution polymer and grafted polymer have been reported (e.g. SI-ATRP of methyl methacrylate or styrene). (Marutani, et al. 2004 Polymer 45 (7), 2231-2235; Koylu, et al. 2009 Macromolecules 42 (22), 8655-8660.)

### Robust surface mineralization on Ti-pSBMA substrates

To test the impact of surface pSBMA polymer brushes on heterogeneous surface mineralization both in terms of surface mineral coverage and mineral bonding affinity, Ti-pSBMA-200 substrates and the unmodified Ti6Al4V control were subjected to a urea thermal decomposition-mediated mineralization process. This mineralization process was driven by a gradual increase of the pH of an acidic aqueous solution of HA by ammonium hydroxide, generated from controlled thermal decomposition of urea, to induce supersaturation of the mineralization solution and subsequent heterogeneous mineral nucleation and growth. (Liu, et al. 2011 Acta Biomater. 7 (9), 3488-3495; Liu, et al. 2013 Biomaterials 34 (10), 2442-54; Song, et al. 2003 J. Am. Chemi. Soc. 125 (5), 1236-1243.)

A surface coverage of scattered calcium-deficient apatite minerals of 32% was observed on the Ti6A14V surface (**FIGs. 6a** **& b**). (Liu, et al. 2011 Acta Biomater. 7 (9), 3488-3495.) In contrast, much denser spherical mineral nodules composed of platelet-like calcium-deficient apatite crystals (Ca/P ratio: 1.27 ± 0.02) with more than doubled surface coverage (71 %) were obtained on the Ti-pSBMA substrates (**FIGs. 6a** **& b**), supporting that the zwitterionic polymer coatings more effectively templated the heterogeneous mineral nucleation and growth. The numerous cationic and anionic residues located along the grafted pSBMA side chains could not only more effectively attract oppositely charged precursor ions than the surface oxides on the unmodified Ti6Al4V, but also significantly increase the potential nucleation sites and more effectively reduce the interfacial free energy for heterogeneous nucleation and mineral growth. (Liu, et al. 2013 Biomaterials 34 (10), 2442-54; Mann, S., Biomineralization: Principles and Concepts in Bioinorganic Materials Chemistry. Oxford University Press: Oxford, 2001.)

Also important, the results showed that the surface minerals formed on Ti-pSBMA exhibited more robust adherence to the metallic substrate than those formed on unmodified Ti6A14V control. Whereas most of the surface minerals on Ti6A14V could not withstand ultrasonication in water, more than half of the surface minerals formed on the Ti-pSBMA-200 substrate were retained upon identical sonication treatment (**FIGs. 6a** **& b**). Previous study on the HA-mineralization of 3-D zwitterionic pSBMA hydrogel revealed that the HA mineral growth was directly templated by the zwitterionic ligands, resulting in the detection of the organic zwitterionic ligands within the mineral nodules (supported by high-resolution transmission electron microscopy and associated elemental analysis of the mineral nodules sectioned by focused ion beam). (Liu, et al. 2013 Biomaterials 34 (10), 2442-54.)

Here, the zwitterionic surface brushes were structurally integrated with the surface minerals as a result of their direct templating role during the surface mineralization, thereby resulting in improved bonding of the surface minerals to the metallic substrate (**FIG. 6d**). The results also showed that the extent of surface mineralization including the strongly adhered surface minerals positively correlated with the length of the grafted pSBMA brushes (degree of polymerization, DPs, **FIG. 6c**), further supporting that the surface zwitterionic motifs directly participated in the templated surface-mineralization (**FIG. 6d**).

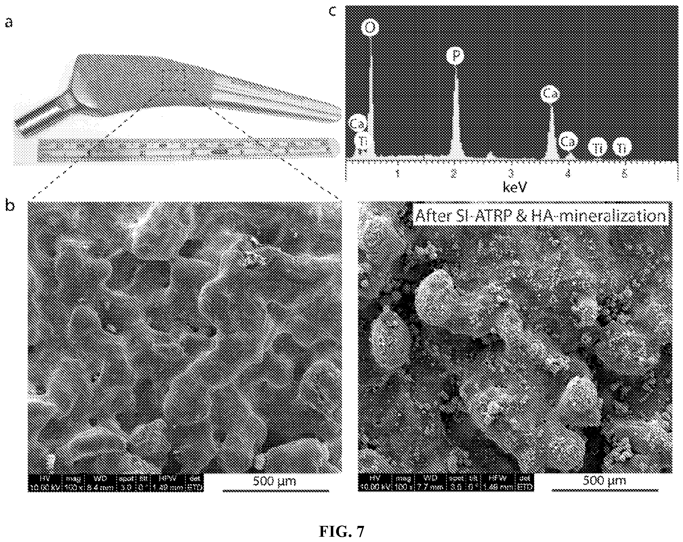

It was also demonstrated that the SI-ATRP of pSBMA and subsequent mineralization could be extended to commercial orthopedic implants with more complex surface topology/porosities. The porous stem region of a Ti6Al4V hip stem (Biomet Taperloc®, Complete Hip Stem, **FIGs. 7a** **& b**) was surface grafted with pSBMA (DP = 200) using identical optimized SI-ATRP conditions. Subsequent mineralization showed substantial calcium-deficient apatite mineral (Ca/P = 1.27 ± 0.14) formed throughout the surface of the macropores (yet without blocking the desired macropores) the implant asrevealed by SEM micrographs (**FIG. 7b**) and the associated EDX spectrum (**FIG. 7c**).

### Cytocompatibility of the surface modifications

To ensure the cytocompatibility of the surface modifications including the grafting of pSBMA and subsequent surface-mineralization, the viability of bone marrow stromal cells cultured in the presence of pristine Ti6A4V, Ti-pSBMA, and mineralized Ti-pSBMA (Ti-pSBMA-min) substrates up to 72 h were evaluated and compared with tissue culture polystyrene (TCPS) control in the absence of any metal substrates. The cells cultured with the pristine Ti6A14V substrates were able to proliferate well, resulting in comparable number of viable cells by 72 h compared to those cultured in the absence of any metallic substrates (**FIG. 8**), agreeing with the well-established cytocompatibility of Ti6A4V. (Bruni, S.; Martinesi, M.; Stio, M.; Treves, C.; Bacci, T.; Borgioli, F., Effects of surface treatment of Ti-6Al-4V titanium alloy on biocompatibility in cultured human umbilical vein endothelial cells. Acta Biomate.r 2005, 1 (2), 223-234.) Neither the presence of the zwitterionic pSBMA coating (Ti-pSBMA), known for its biocompatibility, nor the surface mineralization with osteoconductive calcium apatite (Ti-pSBMA-min) compromised the cellular proliferation over 72 h. There was no significant difference in viable cells compared to those exposed to pristine Ti6Al4V

The facile surface modification strategy demonstrated here was also extended to real orthopedic implants. The enhanced surface mineralization of metallic implants, combined with its demonstrated cytocompatibility and therapeutic delivery of osteoinductive growth factors (e.g., rhBMP-2) via the osteoconductive mineral coating, can improve the osteointegration of the metallic orthopedic and dental implants.

In one aspect, the invention generally relates to a surface layer on a substrate having a structurally integrated mineral grown from a zwitterionic polymer template, wherein the zwitterionic polymer covalently linked to the substrate surface.

In certain embodiments, the zwitterionic polymer has side chains with zwitterionic groups. In certain embodiments, the zwitterionic groups are selected from phosphorylcholine, sulfobetaine, and carboxybetaine. In certain preferred embodiments, the zwitterionic moiety is sulfobetaine.

The sulfobetaine has the structure: 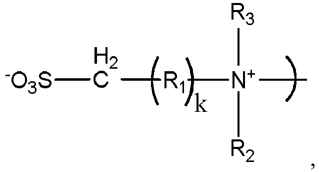 wherein R₁ is -CH₂-, -O-CH₂-, or -O-C₂H₅-, k is an integer from about 0 to about 15, each R₂ and R₃ is independently an alkyl group (*e.g*., methyl, ethyl, other C₁-C₁₂ alkyl groups).

Phosphobetaine has the structure of 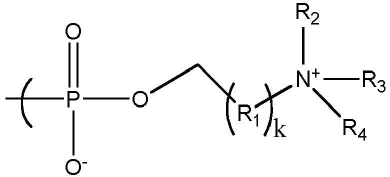 wherein R₁ is -CH₂-, -O-CH₂-, or -O-C₂H₅-, k is an integer from about 0 to about 15, each R₂, R₃ and R₄ is independently an alkyl group (*e.g*., methyl, ethyl, other C₁-C₁₂ alkyl groups).

Carboxybetaine has the structure: 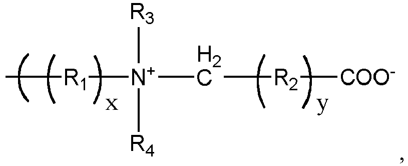 wherein each R₁ and R₂ is independently -CH₂-, -O-CH₂-, or -O-C₂H₅-, x is an integer from about 1 to about 15, y is an integer from about 0 to about 15, each R₃ and R₄ is independently an alkyl group (*e.g.*, methyl, ethyl, other C₁-C₁₂ alkyl groups).

The zwitterionic polymer may be a homopolymer or a copolymer (*e.g*., block or random copolymer). The zwitterionic polymer may have any suitable molecular weight, for example, from about 1,000 Da to about 300,000 Da (*e.g*., from about 1,000 Da to about 200,000 Da, about 1,000 Da to about 100,000 Da, about 1,000 Da to about 50,000 Da about 1,000 Da to about 10,000 Da, about 2,000 Da to about 300,000 Da, about 5,000 Da to about 300,000 Da, about 10,000 Da to about 300,000 Da, about 50,000 Da to about 300,000 Da, about 100,000 Da to about 300,000 Da, about 200,000 Da to about 300,000 Da, about 3,000 Da to about 200,000 Da, about 3,000 Da to about 100,000 Da, about 3,000 Da to about 50,000 Da).

The mineral components may include any suitable material (synthetic or natural) such as bone mineral, *e.g.,* hydroxyapatite, substituted hydroxyapatites (*e.g.,* carbonated, halogenated, metal ion-substituted), calcium deficient hydroxyapatite, calcium apatite, calcium phosphates, octacalcium phosphate, tricalcium phosphate, and any transitional mineral phases between amorphous calcium phosphate to crystalline calcium apatite, and both amorphous and crystalline forms of calcium carbonate. In certain preferred embodiments, the mineral comprises hydroxyapatite.

The mineral components may have any suitable mineral domain morphology. In certain preferred embodiments, the composite material has a mineral domain morphology characterized with isolated mineral nodules (*e.g*., spherical or substantially spherical) having a dimension from about 1 µm to about 300 µm (*e.g*., about 5 µm, 10 µm, 25 µm, 50 µm, 100 µm, 150 µm, 200 µm, 250 µm) (including Type I and Type II mineral domain morphologies).

The surface mineral coverage may be selected according to the application. In certain embodiments, the mineral coverage of the substrate surface is from about 10% to about 100% (*e.g.,* from about 10% to about 98%, from about 10% to about 95%, from about 10% to about 90%, from about 10% to about 80%, from about 20% to about 99%, from about 40% to about 99%, from about 15% to about 100%, from about 25% to about 100%, from about 50% to about 100%, from about 60% to about 100%, from about 70% to about 100%, from about 80% to about 100%, from about 90% to about 100%, from about 95% to about 100%, from about 20% to about 98%, from about 20% to about 95%, from about 20% to about 90%).

The surface layer may be that of any suitable substrate, metallic or non-metallic (*e.g*., ceramic), synthetic or non-synthetic. In certain embodiments, the metallic or ceramic substrate is selected from titanium, stainless steel, cobalt, chromium, tantalum, magnesium, and/or nickel, or an alloy thereof, aluminum oxide and zirconium oxide. In certain embodiments, the metallic substrate comprises Ti6A14V.

The surface layer may be that of a medical implant or a component thereof. The medical implant or component may be polymeric or metallic implants, screws, fixators and surgical devices or a component thereof, such as a dental implant, an orthopedic implant, an percutaneous orthopedic device, an implant of bone, cartilage, tendon, ligament, osteochondral replacement. Exemplary bone, joint, and dental metallic implants include: fixation plates, IM rods, screws, total joint replacement prosthetics; dental filler/composites; spine fusion metallic cages. Exemplary orthopedic implants include: an implant for total knee replacement (TKR), total hip replacement (THR), total shoulder replacement (TSR), total elbow replacement (TER), total wrist replacement (TWR), total ankle replacement (TAR), plates, screws, spine cages, or a component thereof, and the percutaneous orthopedic device is percutaneous screws (e.g. in external fixator).

In certain preferred embodiments, the surface layer is cytocompatible. In certain preferred embodiments, the surface layer is biodegradable.

Further described is a device, or component thereof, having a surface covalently bonded thereto a zwitterionic polymer and a layer of a structurally integrated mineral grown from the zwitterionic polymer as template. The zwitterionic polymer comprises a repeating unit having structure of: 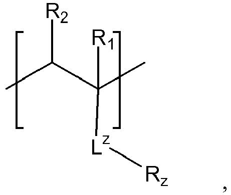 wherein R₁ is a hydrogen, alkyl, alkyloxy; R₂ is a hydrogen, (C₁-C₁₅) alkyl, (C₁-C₁₅) alkyloxy; L^{z} is a linking group; and R_{z} is a pendant group comprising a zwitterionic group.

The zwitterionic polymer can have side chains with zwitterionic groups. The zwitterionic groups can be selected from phosphorylcholine, sulfobetaine, and carboxybetaine. In certain preferred embodiments, the zwitterionic moiety is sulfobetaine.

The zwitterionic polymer can have a repeating unit of structure: 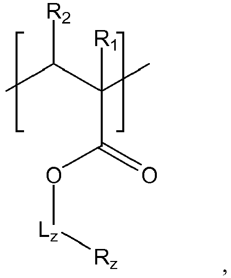 wherein L_{z} is a linking group and R_{z} is a pendant group comprising a zwitterionic group.

R₂ can be hydrogen and R₁ can be hydrogen or methyl group.

L_{z} is -(CH₂)ᵢ-, wherein *i* can be an integer from about 1 to about 20 (*e.g.,* from about 1 to about 15, from about 1 to about 12, from about 1 to about 6, from about 1 to about 3, from about 3 to about 15, from about 3 to about 12, from about 3 to about 9, from about 3 to about 6).

The zwitterionic polymer can be a molecular weight from about 1,000 Da to about 300,000 Da (*e.g*., from about 2,000 Da to about 300,000 Da, from about 5,000 Da to about 300,000 Da, from about 10,000 Da to about 300,000 Da, from about 20,000 Da to about 300,000 Da, from about 50,000 Da to about 300,000 Da, from about 75,000 Da to about 300,000 Da, from about 100,000 Da to about 300,000 Da, from about 150,000 Da to about 300,000 Da, from about 200,000 Da to about 300,000 Da, from about 1,000 Da to about 250,000, from about 1,000 Da to about 200,000, from about 1,000 Da to about 150,000 from about 1,000 Da to about 125,000 Da, from about 100,000 Da to about 75,000 Da, from about 1,000 Da to about 50,000 Da, from about 1,000 Da to about 25,000 Da, from about 1,000 Da to about 10,000 Da, from about 5,000 Da to about 300,000 Da, from about 5,000 Da to about 250,000 Da, from about 10,000 Da to about 250,000 Da, from about 10,000 Da to about 200,000 Da, from about 10,000 Da to about 150,000 Da, from about 10,000 Da to about 100,000 Da, from about 10,000 Da to about 50,000 Da).

In the device, or component thereof, the mineral can be selected from calcium apatites, hydroxyapatite, substituted hydroxyapatites, calcium deficient hydroxyapatite, calcium phosphates, octacalcium phosphate, tricalcium phosphate, and any transitional mineral phases between amorphous calcium phosphate to crystalline calcium apatite, and both amorphous and crystalline forms of calcium carbonate. In certain preferred embodiments, the mineral comprises hydroxyapatite.

In the device, or component thereof, the surface mineral coverage may be selected according to the application. In certain embodiments, the mineral coverage of the substrate surface is from about 10% to about 100% (*e.g*., from about 10% to about 98%, from about 10% to about 95%, from about 10% to about 90%, from about 10% to about 80%, from about 15% to about 100%, from about 20% to about 100%, from about 50% to about 100%, from about 60% to about 100%, from about 70% to about 100%, from about 80% to about 100%, from about 90% to about 100%, from about 95% to about 100%, from about 20% to about 98%, from about 20% to about 95%, from about 20% to about 90%).

The device, or component thereof, may be metallic or non-metallic (*e.g*., ceramic), synthetic or non-synthetic. The substrate can be a metallic material. As used herein, the term "metallic" material refers to a metal, for example, selected from titanium, stainless steel, cobalt, chromium, tantalum, magnesium, and/or nickel, or an alloy thereof, as well as oxides of metals or a metal alloys. The device or component can comprise Ti6A14V.

The substrate can be a ceramic material. As used herein, the term "ceramic" material refers to an inorganic, nonmetallic solid comprising metal, nonmetal or metalloid atoms primarily held in ionic and covalent bonds. Ceramic materials, for example, comprise alumina (aluminum oxide) or zirconia (zirconium oxide).

The device, or component thereof, may be a medical implant or a component thereof. The medical implant or component may be polymeric, or ceramic or metallic implants, screws, fixators and surgical devices or a component thereof, such as a dental implant, an orthopedic implant, an percutaneous orthopedic device, an implant of bone, cartilage, tendon, ligament, osteochondral replacement. Exemplary bone, joint, and dental metallic implants include: fixation plates, IM rods, screws, total joint replacement prosthetics; dental filler/composites; spine fusion metallic cages. Exemplary orthopedic implants include: an implant for total knee replacement (TKR), total hip replacement (THR), total shoulder replacement (TSR), total elbow replacement (TER), total wrist replacement (TWR), total ankle replacement (TAR), plates, screws, spine cages, or a component thereof, and the percutaneous orthopedic device is percutaneous screws (e.g. in external fixator).

Described herein is a method for mineralizing a surface of an object of a metallic or ceramic substrate. The method includes: covalently grafting zwitterionic polymer brushes to a surface of the object of a metallic or ceramic substrate; and templating nucleation and growth of a mineral in the grafted zwitterionic polymer brushes thereby forming a coating of the mineral on the surface of the object.

In method, the object can be a metallic or ceramic implant, or a component thereof.

In the method, the metallic or ceramic implant, or a component thereof, comprises titanium or an alloy thereof. In the method, the metallic or ceramic implant, or a component thereof, can comprise Ti6A14V.

The metallic or ceramic implant, or a component thereof, may be any suitable implant object, for example, a dental implant, an orthopedic implant, or a percutaneous orthopedic device, or a component thereof.

In the method, templating nucleation and growth of a mineral in the grafted zwitterionic polymer brushes can be performed *in vitro.* In the method, templating nucleation and growth of a mineral in the grafted zwitterionic polymer brushes can be performed *in vivo.*

In the method, the object can be an implant for total knee replacement (TKR), total hip replacement (THR), total shoulder replacement (TSR), total elbow replacement (TER), total wrist replacement (TWR), total ankle replacement (TAR), plates, screws, spine cages, or a component thereof, and the percutaneous orthopedic device is percutaneous screws.

For the method, any suitable minerals may be used, for example, calcium apatites, hydroxyapatite, substituted hydroxyapatites, calcium deficient hydroxyapatite, calcium phosphates, octacalcium phosphate, tricalcium phosphate, and any transitional mineral phases between amorphous calcium phosphate to crystalline calcium apatite, and both amorphous and crystalline forms of calcium carbonate. In certain preferred embodiments of the method, the mineral comprises hydroxyapatite. In the method, the mineral can consist essentially of hydroxyapatite. The coating of the mineral on the surface of the object may be a full coating, i.e., of 100% coverage of the intended surface or object. In certain embodiments, the surface mineral coverage of between about 10% to about 100% (*e.g*., from about 10% to about 98%, from about 10% to about 95%, from about 10% to about 90%, from about 10% to about 80%, from about 20% to about 99%, from about 40% to about 99%, from about 15% to about 100%, from about 25% to about 100%, from about 50% to about 100%, from about 60% to about 100%, from about 70% to about 100%, from about 80% to about 100%, from about 90% to about 100%, from about 95% to about 100%, from about 20% to about 98%, from about 20% to about 95%, from about 20% to about 90%).

### Examples

The experimental results demonstrates the preparation of well-controlled zwitterionic pSBMA polymers (PDI < 1.20) prepared through ATRP in an optimized TFE solution containing 10 wt% ionic liquid HMImCl at 60 °C. Equally well-controlled zwitterionic pSBMA brushes (PDI < 1.20) were fabricated via SI-ATRP from Ti6A14V substrates covalently tethered with a phosphonic acid based ATRP initiator, conferring biocompatible and anti-fouling surface properties that are attractive for *in vivo* biomedical applications. The grafted zwitterionic polymer brushes not only effectively templated the surface mineralization, increasing the surface mineral coverage by >100% from those achieved with unmodified Ti6A14V substrate, but also significantly improved the bonding affinity of the surface apatite minerals to the metallic substrate.

### Materials

[2-(Methacryloyloxy)ethyl]dimethyl-(3-sulfopropyl)ammonium hydroxide (SBMA, 97 %), ethyl α-bromoisobutyrate (EBiB, 98 %), α-bromoisobutyryl bromide (BiBB, 98 %), 2, 2'-bipyridyl (BPY, 97 %), copper(I) bromide (CuBr, 99.999 %), 1-hexyl-3-methylimidazolium chloride (HMImCl, 97 %), 2,2,2-trifluoroethanol (TFE, 99 %), bromotrimethylsilane (BTMS, 97 %), anhydrous methanol (99.8 %), anhydrous dichloromethane (DCM, 99.8 %), anhydrous hexane (95 %) and acetone (99.9 %) were purchased from Sigma-Aldrich and used as received. Triethylamine (TEA, 99.5 %, Sigma-Aldrich) was dried by calcium hydride (CaH2, 99.99 %, Sigma-Aldrich) and distilled prior to use. Diethyl (hydroxymethyl)phosphonate (P-OH, 98 %, TCI America) was used as received. Bovine serum albumin (BSA)-fluorescein conjugate was purchased from Invitrogen and used as received. Ti6A14V plate (1.3 mm thick, Titanium Metal Supply Inc.) was cut into 10 × 10 mm² square pieces or 4 × 40 mm² stripe, which were sequentially polished under water with 600, 1500, and 3000 grit silicon carbide sandpapers and ultrasonically cleaned with hexane (10 min), DCM (10 min), and acetone (10 min) sequentially. After the extensive washing, the substrates were annealed in a 120 °C oven prior to use. The porous region of a commercial Ti6A14V hip stem (Taperloc®, Complete Hip Stem, BioMet) was cut into 10 × 10 × 10 mm³ cubic pieces, and treated in the same manner as described for the Ti6A14V plates except that no polishing was carried out with the porous stem surface.

### General Instrumentation

¹H NMR (400 MHz), ¹³CNMR (100 MHz), and ³¹P NMR (170 MHz) spectra were recorded in methanol-d₄ on a Varian INOVA-400 spectrometer. The proton and carbon signals of TMS were used as internal reference for ¹H NMR and ¹³C NMR, while phosphoric acid was added for calibration of the ³¹P NMR. Mass spectra were acquired on a Thermo LTQ in a positive ion mode. The samples were dissolved in water (1mg/mL) and diluted in 50% MeOH to 10 µg/mL and infused using a Triversa Nanomate into the Thermo LTQ.

### Synthesis of (diethoxyphosphoryl)methyl 2-bromo-2-methylpropanoate (P-O-Br)

P-OH (1.68 g, 10 mmol) and TEA (1.52g, 15 mmol) were mixed in 20 mL of anhydrous DCM in a dry, two-neck flask equipped with a dropping funnel and sealed with rubber stoppers. The mixture was cooled in an ice bath before 20 mL of DCM solution of BiBB (3.45 g, 15 mmol) was slowly added via the dropping funnel. The esterification reaction (synthetic scheme shown in FIG. 9) proceeded in the ice bath for 1 h and then continued at room temperature for 24 h. After being washed with 10% HCl aqueous solution (4 times, 45 mL each time), the organic phase was dried with anhydrous calcium sulfate and concentrated under reduced pressure yielding a brown oil-like crude product. The product was purified by flash chromatography with ethyl acetate as an eluent to obtain clear oil product (63.6% yield). ¹H NMR (400 MHz, methanol-d₄): δ 4.46 (d, *J* = 8.5 Hz, 2H), 4.18 (m, 4H), 1.94 (s, 6H), δ 1.34 (t, *J* = 7.1 Hz, 6H) ppm; ¹³C NMR (100 MHz, methanol-d₄): δ 170.7, 63.4, 63.4, 58.3, 56.6, 54.9, 29.9, 15.6, 15.6 ppm; ³¹P NMR (170MHz, methanol-d₄): δ 17.8 ppm. MS (ESI, m/z) for C₉H₁₈Br₁O₅P₁: [M + H]+ calculated, 317.09, found 317.11.

### Synthesis of initiator (2-bromo-2-methylpropanoyloxy) methylphosphonic acid (PA-O-Br)

The deprotection of P-O-Br was carried out in a two-step process (synthetic scheme shown in **FIG. 9**). P-O-Br (1.06 g, 3.34 mmol) was dissolved in 20 mL of anhydrous DCM in a dry flask, to which BTMS (1.86 g, 12 mmol) was subsequently added by injection. The reaction proceeded at room temperature under argon atmosphere overnight before volatile components and DCM were removed under vacuum. Anhydrous methanol (15 mL) was then added to the intermediate and stirred at room temperature overnight before the solvent and volatiles were removed under vacuum yielding a yellowish oil. After recrystallization in DCM, the product was obtained as white crystals (47 % yield). ¹H NMR (400 MHz, methanol-d₄): δ 4.39 (dd, *J* = 9.0, 1.6 Hz, 2H), 1.94 (s, 6H) ppm; ¹³C NMR (100 MHz, methanol-d₄): δ 171.1, 60.1, 58.5, 55.2, 29.9 ppm; ³¹P NMR (170MHz, methanol-d₄): δ 14.9 ppm. MS (ESI, m/z) for C₅H₁₀Br₁O₅P₁: [M + H]+ calculated, 261.01, found 261.09. All original NMR and MS spectra are shown in **FIGs. S2-S5.**

### Surface immobilization of initiator PA-O-Br on Ti6Al4V

Annealed Ti6A14V substrates (40 pieces) were cleaned in an air plasma cleaner (Harrick, PDC-001) for 2 min before being placed in a plastic dish, and submerged under 40 mL of 3-mM anhydrous methanol solution of PA-O-Br at room temperature in dark for 24 h to allow the phosphonic acid group to attach to the thin oxidized metallic surface. All retrieved substrates (Ti-Br) were then annealed at 110 °C for 15 min in a vacuum oven, followed by extensive sonication in methanol (10 min each time, twice), and dried under vacuum.

### Optimization of the conditions for ATRP of zwitterionic SBMA

To optimize the ATRP conditions, a series of polymerizations varying the reaction temperature and with/without the introduction of HMImCl (1:10 by weight relative to TFE used⁴⁴) were carried out (**Table 1**). In a typical procedure, BPY (0.2 mmol) was dissolved in TFE in a Schlenk flask and degassed by three "freeze-pump-thaw" cycles to remove oxygen. The flask was then back-filled with argon and CuBr (0.1 mmol) was added into the flask under the argon protection. The mixture was stirred for 10 min to ensure the formation of the copper catalyst complex. Monomer SBMA (10 mmol, for DP = 100), free initiator EBiB (0.1 mmol), and HMImCl were dissolved in TFE under stirring in the second Schlenk flask at room temperature. The flask was then degassed by three "freeze-pump-thaw" cycles, after which the copper catalyst complex was added by syringe. The mixture was allowed to stir for an additional 1 min before the flask was mounted in an isothermal water bath to start the polymerization. During the course of the reaction, aliquots of the reaction mixture were retrieved at various time points for ¹H NMR and GPC monitoring of the ATRP reaction. After the predetermined time, the reactor was exposed to air to terminate the polymerization and the resulting mixture was precipitated in methanol to obtain free zwitterionic pSBMA polymer.

### Grafting pSBMA polymer brushes from Ti-Br surfaces by SI-ATRP

The SI-ATRP was conducted under the optimized conditions (with the introduction of HMImCl and under 60°C). The process was similar to that of the solution ATRP described above. Instead of conducting the polymerization in the second Schlenk flask, the mixture was quickly transferred into a flat-bottom reactor containing Ti-Br substrates under argon atmosphere after the 1 min's stirring, ensuring the SI-ATRP and solution ATRP were almost simultaneously progressed. When the polymerization was completed, the pSBMA-grafted Ti6A14V substrates (e.g. Ti-pSBMA-100, where 100 refers to the targeting degree of polymerization, DP) were extracted with TFE using a Soxhlet apparatus for 24 h to remove the free polymer physically absorbed on the surface, and dried under vacuum. pSBMA brushes with different targeting DPs (50, 200) were grafted from Ti-Br by varying the ratio of monomers relative to initiators accordingly.

### Torsion test

To evaluate the bulk mechanical property of the Ti substrates before and after modification, torsion tests of Ti6A14V and Ti-pSBMA substrates (1.3 × 4 × 40 mm³ stripes, n = 3) were performed on an MTS Bionix 370 test system equipped with a 150 kN/150 N-m load cell from 0 to 70° at 0.2°/s at room temperature. Torsional stiffness of the substrates was calculated from the linear region (0 to 15°) of the respective torque-displacement curves.

### Cleavage of pSBMA brushes from the pSBMA-grafted (Ti-pSBMA) substrates

The Ti-pSBMA substrates were placed in a 50-mL plastic Corning tube containing 30 mL of ionic acidic cleaving solution (0.2 M NaCl and 2 M HCl aqueous solution), and subjected to gentle shaking on an orbital shaker at room temperature for 72 h. The cleavage solution was then collected, neutralized by sodium hydroxide, and desalted in a dialysis membrane tubing (Spectra/Por 6, MWCO: 1000) against Milli-Q water for 72 h, with regular change of fresh Milli-Q water every 8 h. Cleaved pSBMA was obtained after freeze-drying for subsequent analyses.

### Aqueous gel permeation chromatography (GPC)

GPC of solution polymers or those cleaved from the substrates were performed on a Varian ProStar HPLC system connected with two PL Aquagel-OH columns (type 40 first, followed by type 20, 8 mm, 300×7.5mm, Agilent Technologies) and equipped with a refractive index detector (Varian 356-LC, 35 °C). The eluent was 0.05 M Trisma buffer (pH 7.0) containing 0.2 M NaNO₃ and a flow rate of 1.0 mL/min was applied. Weight- and number-averaged molecular weights (M_{w} and Mₙ) and polydispersity index (PDI) of the polymers were calculated by Cirrus AIA GPC software. Ten narrowly dispersed PEO standards from PL2070-0100 and PL-2080-0101 kits (Polymer Laboratories, Agilent Technologies) were used as calibration standards.

### X-ray photoelectron spectroscopy (XPS)

Surface compositional analyses of substrates before and after SI-ATRP were carried out on an ESCA SSX-100 equipped with an AlK*_{α}* radiation source under the pass energy of 25 eV and the spot size of 600 µm. Survey scan spectra were obtained from two consecutive scans of a randomly chosen area of interest while high resolution scan spectra were obtained from eight consecutive scans. All binding energies were referenced to the C₁ₛ hydrocarbon peak at 285.0 eV.

### Water contract angle measurements

The static water contact angles of the substrates before and after surface modifications were recorded on a CAM200 goniometer (KSV Instruments). A droplet (2 µL) of Milli-Q water was placed on the substrate and the contact angles (left and right) of the droplet were recorded after 30 s. The left and right contact angles of each droplet, and three substrates of each sample group were averaged and reported as averages ± standard deviation.

### Non-specific protein adsorption on surfaces

Ti6A14V substrates with or without grafted pSBMA were placed in a 24-wells culture plate containing 1 mL of BSA-fluorescein conjugate/DPBS solution (500 µg/mL) in each well, and incubated at 37 °C overnight. All substrates were rinsed with fresh DPBS (pH=7.4) three times upon retrieval to move loosely absorbed BSA. The substrate surfaces were then imaged on a Zeiss inverted stage fluorescent microscope. The fluorescence intensities were quantified by ImageJ using line plots.

### Surface mineralization

Mineralization was carried out by controlled heating of the Ti6A14V substrates with or without grafted pSBMA in a urea-containing, acidic solution of hydroxyapatite from 37 °C to 95 °C (with the thermal decomposition of urea gradually causing pH increases, resulting in supersaturation of the mineralization solution) using a protocol modified over a previous report. (Lee, et al. 1998 Polym. Gels Netw. 6 (6), 493-511.) The advantage of this heterogeneous surface-mineralization method over other more commonly used mineralization systems such as those based on simulated body fluids (SBF) was previously demonstrated. (Liu, et al. 2011 Acta Biomater. 7 (9), 3488-3495.) The pSBMA was able to maintain its zwitterionic nature throughout the pH range of this urea thermal decomposition-mediated mineralization process (stable pH-dependent zeta potentials from pH 3 to 9). (Liu, et al. 2013 Biomaterials 34 (10), 2442-54.) Mineralization stock solution was prepared by suspending hydroxyapatite (7.37 g, 34-40% Calcium content, Alfa Aesar) in 500-mL aqueous solution of urea (2 M), followed by the addition of concentrated hydrochloric acid under constant stirring until a clear soluble solution was obtained (final pH 2.5-3.0). Six to ten Ti substrates were placed in an Erlenmeyer flask containing 30 mL of the mineralization solution and covered with a perforated aluminum foil. The flask was placed in a high-temperature silicone oil bath with the mineralization solution completely submerged under the oil and heated by a 100-watt immersion heater (Glo-Quartz LHP-IAH4) equipped with a programmable temperature controller (Eurotherm 2408). Controlled heating from 37.0 °C to 95.0 °C was carried out at a heating rate of 0.2 °C/min. Mineralized substrates were bath-sonicated for 1 min in Milli-Q water to ensure the removal of loosely bound mineral precipitates, and dried under vacuum before further use or characterizations.

### Quantification of surface calcium content

Total calcium content of the mineralized substrates was determined by quantifying the Ca²⁺ ions released (n = 3) from the substrate upon treatment in a hydrochloric acid solution with a Thermo Scientific calcium ion selective electrode attached to a VWR Symphony pH/ISE meter. In a typical procedure, the mineralized substrate was placed in 10 mL of hydrochloric acid solution (pH 3) in a 20-mL glass vial and the pH was adjusted by concentrated hydrochloric acid to around 2.1. The mineral was allowed to be fully released from the substrate under constant shaking of the acidic solution on an orbital shaker. Ionic Strength Adjustment buffer (ISA, 4 M KCl solution, VWR, 200 *µ*L) was added to the acidic solution containing the released calcium prior to measurement by the calcium ion selective electrode. The total calcium content of each type of mineralized substrate was determined using a standard curve generated by a series of acidic (pH 2.1) aqueous Ca²⁺ ion standard solutions containing 0.1, 0.01, 0.001, and 0.0001 M CaCl₂.

**Scanning electron microscopy (SEM) and associated energy dispersive X-ray spectroscopy (EDS).** The morphology of the dried mineralized substrate, coated with 3-nm carbon, was observed on a Quanta 200 FEG MKII scanning electron microscope (SEM, FEI) under an accelerating voltage of 5 or 10 kV, with a spot size of 3.0 µm (aperture 6) and a working distance about 10 mm. EDS was acquired under 10 kV, with a spot size of 3.0 µm. Reported calcium to phosphorus ratios (Ca:P) were calibrated against single crystal HA whiskers prepared by molten salt synthesis (Ca:P = 1.67). (Tas, et al. 2001 J. Am. Ceram. Soc. 84 (2), 295-300.)

**Cell viability and proliferation assay.** To evaluate the cytocompatibility of the surface coatings (pSBMA brushes and the surface mineralization), the viability of rat bone marrow-derived stromal cells (rMSCs) cultured in the presence of Ti6Al4V, Ti-pSBMA, and the mineralized Ti-pSBMA substrates were tested by CCK-8 cell proliferation kit (Dojindo). The Ti6Al4V, Ti-pSBMA, and mineralized Ti-pSBMA substrates (n = 3) were sterilized in ethanol (15 min immersion), and air-dried in a ventilated tissue culture hood. The substrates pre-equilibrated in sterile PBS (pH 7.4) and cell culture medium (a-MEM with 20% FBS, 1% penicillin, and 1% streptomycin, 2% glutamine) were transferred into 24-well culture plate containing 1 mL of fresh medium in each well, to which rMSC (passage 1) suspension (10 µL, 20,000 cells) was added and cultured for up to 72 h. Comparing to directing seeding of cells on metallic surfaces only, this chosen method of cell seeding ensures comparable overall number of adherent cells in each well regardless of the nature of the metallic surfaces (e.g. pSBMA-grafted surfaces are known for reduced cell adhesiveness), thereby ensuring fair comparison of substrate cytocompatibility. Comparing to the other alternative of placing metallic substrates over an established adherent MSC culture, this method also ensures that the access to nutrients and the proliferation of cells are not impeded. At 24 or 72 h after initial cell seeding, cell culture medium in each well was replaced with 0.4 mL of fresh medium along with 40 µL CCK-8 reagents and incubated for 4 h. The absorbance of the removed culture media was read at 450 nm (background subtraction at 620 nm) on a microplate reader.

In this specification and the appended claims, the singular forms "a," "an," and "the" include plural reference, unless the context clearly dictates otherwise.

Unless defined otherwise, all technical and scientific terms used herein have the same meaning as commonly understood by one of ordinary skill in the art. Although any methods and materials similar or equivalent to those described herein can also be used in the practice or testing of the present disclosure, the preferred methods and materials are now described. Methods recited herein may be carried out in any order that is logically possible, in addition to a particular order disclosed.

## Claims

1. A surface layer on a substrate comprising structurally integrated minerals grown from a zwitterionic polymer template, wherein the zwitterionic polymers are covalently linked to the substrate surface, wherein
the substrate is a metallic or ceramic substrate; and
the substrate is **characterized by** having a porous surface.

2. The surface layer of Claim 1, wherein the zwitterionic polymer comprises side chains with zwitterionic groups, wherein the zwitterionic groups are preferably selected from phosphorylcholine, sulfobetaine, and carboxybetaine.

3. The surface layer of Claim 1 or 2, wherein the mineral is selected from calcium apatites, hydroxyapatite, substituted hydroxyapatites, calcium deficient hydroxyapatite, carbonated calcium apatites, calcium phosphates, octacalcium phosphate, tricalcium phosphate, and any transitional mineral phases between amorphous calcium phosphate to crystalline calcium apatite, and both amorphous and crystalline forms of calcium carbonate.

4. The surface layer of any of Claims 1-3, wherein surface mineral coverage is from 10% to 100%.

5. The surface layer of any of Claims 1-4, wherein the metallic substrate is preferably selected from titanium, stainless steel, cobalt, chromium, tantalum, magnesium, and/or nickel, or an alloy thereof, and the ceramic substrate is selected from aluminum oxide and zirconium oxide.

6. The surface layer of any of Claims 1-5, wherein the substrate is a medical implant or a component thereof, wherein the medical implant or component thereof is preferably a dental implant, an orthopedic implant, or a percutaneous orthopedic device.

7. The surface layer of any of Claims 1-6, wherein the surface layer is cytocompatible.

8. The surface layer of any of Claims 1-6, wherein the surface layer is biodegradable.

9. The surface layer of Claim 4, wherein the surface metal coverage is from 50 to 100%.

## Patentansprüche

1. Oberflächenschicht auf einem Substrat, umfassend strukturell integrierte Mineralien, die aus einer zwitterionischen Polymervorlage gewachsen sind, wobei die zwitterionischen Polymere kovalent an die Substratoberfläche gebunden sind, wobei
das Substrat ein metallisches oder keramisches Substrat ist; und
das Substrat **dadurch gekennzeichnet ist, dass** es eine poröse Oberfläche hat.

2. Oberflächenschicht nach Anspruch 1, wobei das zwitterionische Polymer Seitenketten mit zwitterionischen Gruppen umfasst, wobei die zwitterionischen Gruppen vorzugsweise aus Phosphorylcholin, Sulfobetain und Carboxybetain ausgewählt sind.

3. Oberflächenschicht nach Anspruch 1 oder 2, wobei das Mineral ausgewählt ist aus Calciumapatiten, Hydroxyapatit, substituierten Hydroxyapatiten, calciumarmen Hydroxyapatit, kohlensäurehaltigen Calciumapatiten, Calciumphosphaten, Octacalciumphosphat, Trikalciumphosphat und jeglichen mineralischen Übergangsphasen zwischen amorphem Calciumphosphat und kristallinem Calciumapatit sowie sowohl amorphen als auch kristallinen Formen von Calciumcarbonat.

4. Oberflächenschicht nach einem der Ansprüche 1-3, wobei die Mineralbedeckung der Oberfläche 10 % bis 100 % beträgt.

5. Oberflächenschicht nach einem der Ansprüche 1-4, wobei das metallische Substrat vorzugsweise aus Titan, rostfreiem Stahl, Kobalt, Chrom, Tantal, Magnesium und/oder Nickel oder einer Legierung davon ausgewählt ist und das keramische Substrat aus Aluminiumoxid und Zirkoniumoxid ausgewählt ist.

6. Oberflächenschicht nach einem der Ansprüche 1-5, wobei das Substrat ein medizinisches Implantat oder eine Komponente davon ist, wobei das medizinische Implantat oder die Komponente davon vorzugsweise ein Zahnimplantat, ein orthopädisches Implantat oder eine perkutane orthopädische Vorrichtung ist.

7. Oberflächenschicht nach einem der Ansprüche 1-6, wobei die Oberflächenschicht zytokompatibel ist.

8. Oberflächenschicht nach einem der Ansprüche 1-6, wobei die Oberflächenschicht biologisch abbaubar ist.

9. Oberflächenschicht von Anspruch 4, wobei die Oberflächenmetallbedeckung von 50 bis 100% beträgt.

## Revendications

1. Couche de surface sur un substrat comprenant des minéraux intégrés structurellement, cultivés à partir d'une matrice de polymère zwitterionique, dans laquelle les polymères zwitterioniques sont liés de manière covalente à la surface du substrat,
le substrat étant un substrat métallique ou céramique ; et
le substrat étant **caractérisé par** une surface poreuse.

2. Couche de surface selon la revendication 1, le polymère zwitterionique comprenant des chaînes latérales avec des groupes zwitterioniques, les groupes zwitterioniques étant de préférence choisis parmi la phosphorylcholine, la sulfobétaïne et la carboxybétaïne.

3. Couche de surface selon la revendication 1 ou 2, le minéral étant choisi parmi les apatites de calcium, l'hydroxyapatite, les hydroxyapatites substituées, l'hydroxyapatite déficiente en calcium, les apatites de calcium carbonatées, les phosphates de calcium, le phosphate octacalcique, le phosphate tricalcique, et toutes les phases minérales de transition entre le phosphate de calcium amorphe et l'apatite de calcium cristallin, et les formes amorphes et cristallines du carbonate de calcium.

4. Couche de surface selon l'une quelconque des revendications 1 à 3, la couverture minérale superficielle étant de 10 à 100 %.

5. Couche de surface selon l'une quelconque des revendications 1 à 4, le substrat métallique étant de préférence choisi parmi le titane, l'acier inoxydable, le cobalt, le chrome, le tantale, le magnésium et/ou le nickel, ou un alliage de ceux-ci, et le substrat céramique étant choisi parmi l'oxyde d'aluminium et l'oxyde de zirconium.

6. Couche de surface selon l'une quelconque des revendications 1 à 5, le substrat étant un implant médical ou un composant de celui-ci, l'implant médical ou le composant de celui-ci étant de préférence un implant dentaire, un implant orthopédique ou un dispositif orthopédique percutané.

7. Couche de surface selon l'une quelconque des revendications 1 à 6, la couche de surface étant cytocompatible.

8. La couche de surface selon l'une quelconque des revendications 1 à 6, la couche de surface étant biodégradable.

9. La couche de surface de la revendication 4, la couverture métallique de surface étant de 50 à 100 %.
